# EUROPÄISCHE PATENTSCHRIFT

(11) **EP 2 442 758 B1**
(45) Veröffentlichungstag und Bekanntmachung des Hinweises auf die Patenterteilung: **29.05.2013**
(21) Anmeldenummer: 10725985.5
(22) Anmeldetag: 03.06.2010
(51) Int. Cl.: A61F 2/72, A61F 4/00

(54) **AUSSENOHRMUSKULATURERFASSUNGSMITTEL**
DEVICE FOR CAPTURING MUSCLE ACTIVITY OF THE OUTER EAR
DISPOSITIF POUR DÉTECTER L'ACTIVITÉ MUSCULAIRE DE L'OREILLE EXTERNE

(30) Priorität: 15.06.2009 DE 102009025313
(43) Veröffentlichungstag der Anmeldung: 25.04.2012
(73) Patentinhaber: Georg-August-Universität Göttingen Stiftung Öffentlichen Rechts, 37073 Göttingen (DE)
(72) Erfinder: LIEBETANZ, David, 37073 Göttingen (DE)
(74) Vertreter: Günther, Constantin
(86) Internationale Anmeldenummer: PCT/EP2010/003367
(87) Internationale Veröffentlichungsnummer: WO 2010/145759

(56) Entgegenhaltungen:
- EP-A2- 0 468 340
- EP-A2- 1 779 820
- WO-A1-2004/034937
- US-A1- 2005 085 744
- US-A1- 2005 195 155

## Beschreibung

Die Erfindung betrifft die Erfassung einer Aktivierung zumindest eines Teils der äußeren Ohrmuskulatur eines Lebenswesens.

Ein technisches Anwendungsgebiet der Erfindung liegt beispielsweise in der Neuroprothetik. Hier werden verbesserte Möglichkeiten gesucht, um Signale des Nervensystems abzuleiten, welche willkürlich erzeugt oder modifiziert werden können. Durch solche abgeleiteten Signale sollen beispielsweise technische Hilfssysteme für ausgefallene Körperfunktionen (Prothesen) gesteuert werden.

Eine solche Prothesensteuerung ist z.B. aus der DE 101 24 839 A1 bekannt. Danach besteht eine technische Möglichkeit zur Steuerung von Prothesen darin, Muskelsignale aus der verbliebenen Muskulatur amputierter Extremitäten abzuleiten. Die Signale können als EMG-Signale oder, wie in der DE 101 24 839 A1 vorgeschlagen, mittels einer optischen Sensierung erfasst werden.

Beispielsweise können die Muskelsignale aus der Schultermuskulatur abgeleitet werden. Durch die Technik der targeted muscle reinnervation können die Nervenstümpfe der amputierten Extremität die proximale Muskulatur aktivieren, d.h. reizen. Die hiervon erhaltenen EMG-Signale entsprechen der Aktivierung der amputierten Extremität. Hierdurch kann eine Prothesensteuerung basierend auf den originären Nervenimpulsen der amputierten Extremität erfolgen. Allerdings hat diese Technik den Nachteil, dass ein relativ umfangreicher invasiver Eingriff mit den entsprechenden Risiken erforderlich ist. Zudem ist die Anwendbarkeit auf amputierte Extremitäten begrenzt. Bei gelähmten Extremitäten kann diese Technik nicht angewendet werden.

Eine Hilfseinrichtung für ein Lebewesen mit einer Steuereinrichtung zur Steuerung eines technischen Hilfsmittels ist z. B. aus der WO 2004/034937 A1, US 2005/195155 A1, EP 0 468 340 A2, US 2005/085744 A1 oder EP 1 779 820 A2 bekannt.

Der Erfindung liegt daher die Aufgabe zugrunde, Möglichkeiten zur Steuerung von Hilfsmitteln und anderen Einrichtungen aufzuzeigen, die einfacher und weniger invasiv am Menschen einsetzbar sind.

Diese Aufgabe wird durch eine Hilfseinrichtung für ein Lebewesen mit einem Außenohrmuskulaturerfassungsmittel gelöst, das eine Aktivierung zumindest eines Teils der äußeren Ohrmuskulatur eines Lebewesens erfasst. Die Hilfseinrichtung weist eine mit dem Außenohrmuskulaturerfassungsmittel verbundene Steuereinrichtung auf. Die Steuereinrichtung ist zur Verarbeitung der von dem Außenohrmuskulaturerfassungsmittel erfassten Signale und zur Ausgabe von Steuerungssignalen an ein technisches Hilfsmittel eingerichtet. Das technische Hilfsmittel ist über die Steuerungssignale steuerbar. Auf diese Weise können grundsätzlich beliebige technische Hilfsmittel durch die erfindungsgemäße Hilfseinrichtung gesteuert werden. Unter einer Muskelaktivierung ist in diesem Zusammenhang jede Muskelbetätigung im weitesten Sinne zu verstehen, insbesondere auch geringfügige Muskelbetätigungen, die nicht unbedingt zu äußerlich sichtbaren Muskelkontraktionen führen.

Das menschliche Ohr gliedert sich in das Außenohr, das Mittelohr und das Innenohr. Im Mittelohr eines Menschen befinden sich bekanntlich die Gehörknöchelchen Hammer, Amboss und Steigbügel. Zudem sind in diesem Mittelohrbereich Muskeln vorhanden, wie z.B. Trommelfellspannmuskel und der Stapediusmuskel, die sich bei Wahrnehmung eines hohen Schallpegels unwillkürlich reflexartig anspannen. Durch diese reflexartige Muskelanspannung wird das Gehör vor Schäden durch zu hohe Schallpegel geschützt. Die reflexartige An- /spannung des Stapediusmuskels, auch als Stapediusreflex bezeichnet, wird z.B. im Bereich von Cochleaimplantaten zur künstlichen Wiederherstellung des Hörvermögens genutzt, wie z.B. in der DE 10 2007 008 154 A1 beschrieben. Die zuvor genannten reflexartigen Muskelreaktionen im Bereich des Mittel- und Innenohrs sind vom Menschen nicht willkürlich beeinflussbar und eignen sich daher nicht für die Ableitung von Signalen beispielsweise zur Prothesensteuerung. Die vorliegende Erfindung beruht auf der Erkenntnis, dass der Mensch auch im Bereich des Außenohrs Muskeln zur Verfügung hat - nachfolgend als äußere Ohrmuskulatur bezeichnet - die jedoch bisher keine Beachtung fanden, da sie im Normalfall nicht genutzt werden. Beim Menschen sind an jedem Ohr neun äußere Ohrmuskeln vorhanden. Es wurde herausgefunden, dass diese Muskeln mit entsprechendem Training vom Menschen willkürlich aktivierbar sind.

Im Gegensatz zur übrigen Fazialis-innervierten Muskulatur der mimischen Muskulatur besitzt die äußere Ohrmuskulatur keine wesentliche Funktion beim Menschen. Daher ist die äußere Ohrmuskulatur normalerweise nur sehr eingeschränkt der Willkürmotorik unterworfen. Jedoch handelt es sich dabei um quergestreifte Muskulatur, die vom Nervus fazialis innerviert wird. Dementsprechend schlägt die vorliegende Erfindung technische Möglichkeiten vor, Signale aus der äußeren Ohrmuskulatur abzuleiten und zu verwenden, z.B. für die Steuerung von Prothesen oder anderen technischen Geräten, sowohl für behinderte als auch für nicht behinderte Personen.

Die Heranziehung der äußeren Ohrmuskulatur hat den Vorteil, dass die Muskeln im Wesentlichen von außen zugänglich sind und eine Anbringung von Sensormitteln, z.B. Elektroden, nicht-invasiv oder zumindest wenig invasiv erfolgen kann und somit risikoarm ist. Denkbar ist z.B., ein Außenohrmuskulaturerfassungsmittel mit Elektroden zur Erfassung der Muskelbetätigung mit einem Aufsteckclip retroaurikulär, d.h. hinter der Ohrmuschel, am Ohr des Menschen zu befestigen, so dass das Außenohrmuskulaturerfassungsmittel jederzeit abgenommen werden kann. Das erfindungsgemäße Außenohrmuskulaturerfassungsmittel ist somit besonders anwenderfreundlich und kann zudem einfach und preisgünstig hergestellt werden.

Gegenüber Techniken, die im Bereich der Brain-Computer-Interface (BCI)-Technologie entwickelt wurden, hat die Erfindung den Vorteil einer geringeren Fehleranfälligkeit der Signalerfassung. Bei der BCI-Technologie ist die Fehleranfälligkeit der Signalerfassung aufgrund der Verwendung einer relativ großen Anzahl von Oberflächenelektroden, die zudem wenig stabil am Körper befestigbar sind, relativ hoch. Zudem ist nur eine relativ niedrige Datenrate möglich. Es hat sich auch herausgestellt, dass es für Patienten schwierig ist, die willkürliche Beeinflussung der Hirnströme zu erlernen. Ein weiterer Nachteil besteht darin, dass Patienten während der Verwendung eines BCI keine anderen Tätigkeiten parallel durchführen können, da dies die Hirnströme anderweitig beeinflusst und die Signalqualität reduziert.

Im Gegensatz hierzu bietet die Erfindung den Vorteil, dass Körperelemente des Lebewesens verwendet werden können, die einerseits relativ gut zugänglich und hinsichtlich der willkürlichen Betätigung trainierbar sind, andererseits nicht für sonstige Alltagstätigkeiten benötigt werden. Durch die relativ hohe Zahl zur Verfügung stehender Muskeln am Außenohr, nämlich neun pro Ohr, kann im Maximalfall eine 18-kanalige Steuerung von Hilfsmitteln erfolgen, ohne dass andere Tätigkeiten dabei eingeschränkt werden müssen.

Zum Training der Muskeln am Außenohr wurde eine Reihe von spezifischen Verfahren entwickelt, welche die äußere Ohrmuskulatur in einem ausreichenden Maße der Willkürmotorik zugänglich machen, damit mehrere der äußeren Ohrmuskeln unabhängig voneinander willkürlich aktiviert werden können. Dies ermöglicht erst eine vergleichsweise hohe Anzahl von Steuerkanälen (1 bis maximal 9 pro Seite) für das System. Die Verfahren dienen der Willkürrekrutierung der äußeren Ohrmuskulatur durch neuroplastische Reorganisation der motokortikalen Repräsentation. Die hierfür entwickelten Verfahren können als ganze Sequenz, in Kombination oder auch nur einzeln zur Anwendung kommen.
A) Die kortikale Repräsentation der Fazialis-innervierten Muskulatur inklusive der äußeren Ohrmuskulatur wird mit Hilfe der transkraniellen Magnetstimulation (TMS) und/oder mittels funktioneller Kernspintomographie kartiert.
B) Mittels gepaarter assoziativer Stimulation und transkranieller Gleichstromstimulation wird die kortikale Erregbarkeit vor und während der Trainingsphase (nachfolgend in D) und E) erläutert) fokal angehoben. Nach in A) das kortikale Repräsentationsareal der einzelnen äußeren Ohrmuskeln im individuellen Patienten kartiert wurde, wird über das jeweilige Areal eine Serie von TMS-Pulsen appliziert, denen jeweils im zeitlichen Abstand ein elektrischer Impuls über dem Nervus fazialis vorausgeht. Hierdurch wird eine gezielte Anhebung der Erregbarkeit der kortikalen Neurone erreicht, welche die äußeren Ohrmuskeln aktivieren. Durch die hierdurch bedingte Erniedrigung der Erregungsschwelle können diese Muskeln nun einfacher und gezielter willkürlich aktiviert werden.
C) Nach Amputation einer oberen Extremität dehnt sich die faziale Repräsentation in das ehemalige Handareal aus. Dies kann zu Misperzeptionen im Phantomglied durch Gesichtsberührung führen (Birbaumer et al., 1997). Auch tierexperimentell konnte gezeigt werden, dass Nervenläsionen eine rasche Expansion von intakten Motorepräsentationen in kortikale Areale induzieren, welche von der Peripherie diskonnektiert sind (Sanes et al., 1988; Donoghue et al., 1990). Durch Aufhebung der lokalen Inhibition und Veränderungen der synaptischen Effektivität werden hierbei latente kortikokortikale Verbindungen reaktiviert (Jacobs und Donoghue, 1991). Interessanterweise zeigt der diskonnektierte Kortex eine erhöhte Modifizierbarkeit durch ansonsten unterschwellige Stimulationen (Ziemann et al., 1998).

Auf diesen neurowissenschaftlichen Prinzipien basierend wird in einem weiteren Verfahren eine transiente Umgebungsdenervierung mittels lokalen Injektionen von Botulinumtoxin in Anteile der fazialen Muskulatur erzielt, welche die äußeren Ohrmuskeln sowie eine Reduktion der synaptischen Effektivität der injizierten Muskulatur erreicht. Hierdurch kommt es zu einer Ausdehnung und Demaskierung der kortikalen Ohrmuskelrepräsentation. Dieser Effekt hält über wenige Monate an. In dieser Zeit sollen dann die übrigen Verfahren (A, B, D und E) zur Anwendungen kommen, um eine anhaltende Willkürkontrolle der äußeren Ohrmuskulatur zu erreichen.
D) Wichtigstes Verfahren der gezielten Willkürrekrutierung ist das Training unter EMG Feedback mit visuellem und/oder akustischem Feedback, z.B. unterstützt durch eine auf einem Computer ablaufende Trainingssoftware.
E) Das Training unter EMG Feedback kann durch ein mechanosensorisches Feedback in der Effektivität noch gesteigert werden. Hierbei wird der zu trainierende einzelne äußere Ohrmuskel analog zum visuellen oder akustischen Feedback während der Muskelaktivierung mechanisch mit einem Vibrator oder einem elektrischen Stimulus in einer Frequenz von 2 bis 50 Hz von außen stimuliert. Hierdurch kommt es über Mechanorezeptoren von Haut und Muskulatur zu einer direkten afferenten Rückkopplung der Muskelaktivität und zu einer Stabilisierung der erniedrigten Aktivierungsschwelle.

Ein weiterer Vorteil der Erfindung besteht darin, dass mit der Nutzung der EMG-Signale der äußeren Ohrmuskulatur für die Steuerung von Prothesen oder technischen Hilfsmitteln der Rehabilitation Patienten mit unterschiedlichsten Lähmungs- und Amputationsmustern bis hin zu einer sehr hohen zervikalen Querschnittslähmung versorgt werden können. Vorteilhaft wird die Erkenntnis ausgenutzt, dass auch Querschnittsgelähmte in der Regel die äußere Ohrmuskulatur aktivieren können. Ein weiterer wesentlicher Vorteil besteht darin, dass das System unabhängig von anderen Aktivitäten verwendet werden kann (z.B. auch beim Laufen oder Sprechen). Darüber hinaus ist für die Implantation von epimysialen EMG-Elektroden oder einer Verstärker/Transmitter-Einheit nur ein minimal invasiver risikoarmer Eingriff notwendig, im Gegensatz z.B. zur Implantation von subduralen Elektroden oder zu den größeren Eingriffen im Rahmen der targeted muscle reinnervation.

Das Außenohrmuskulaturerfassungsmittel kann im Einzelnen unterschiedlich ausgebildet sein. Entscheidend ist, dass eine Aktivierung zumindest eines Teils der äußeren Ohrmuskulatur eines Lebewesens, also eines Menschen oder Tieres, erfasst wird.

Gemäß einer vorteilhaften Ausgestaltung weist das Außenohrmuskulaturerfassungsmittel ein Sensormittel zur Erfassung der elektrischen Muskelaktivität wenigstens eines äußeren Ohrmuskels auf. Die Erfassung eines elektrischen Signals am Körper des Menschen hat den Vorteil, dass relativ einfach aufgebaute Elektroden verwendet werden können und keine sonstigen, komplizierteren Apparaturen erforderlich sind. Vorteilhaft stehen außerdem gleich elektrische Signale zur Verfügung, die elektrisch verstärkt und weiterverarbeitet werden können. Vorteilhaft können als Sensormittel z.B. EMG-Elektroden verwendet werden. EMG ist eine Abkürzung für die Elektromyographie, die in der Humanmedizin eine elektrophysiologische Methode der Diagnostik in der Neurologie ist, bei der die elektrische Muskelaktivität gemessen wird. Mit Hilfe von Nadelelektroden lassen sich beispielsweise Potentialschwankungen einzelner Muskeln ableiten. Mit Spezialnadeln lassen sich auch einzelne Muskelfasern erfassen. Die Sensormittel umfassen jedoch auch als Oberflächenelektroden ausgebildete EMG-Elektroden, die Signale durch Messung von Potentialänderungen auf der Haut erzeugen. Mit solchen EMG-Signalen wird die elektrische Aktivität des Muskels je nach Stärke der Muskelkontraktion erfasst. Es sind somit abgestufte Signale je nach Stärke der Muskelkontraktion ableitbar. Für eine chronische Ableitung von EMG-Signalen können intra- oder epimysiale Elektroden verwendet werden, die entsprechend unter die Haut implantiert werden.

In einer weiteren Ausgestaltung weist das Außenohrmuskulaturerfassungsmittel ein Sensormittel zur Erfassung einer durch einen Ohrmuskel ausgelösten mechanischen Bewegung auf. Geeignete Sensormittel hierfür sind z.B. Kraftmesssensoren, wie Piezo-Sensoren oder Dehnungsmessstreifen, oder optische Sensoren, wie aus der DE 101 24 839 A1 bekannt. Hierfür kann an der im Bereich des zu erfassenden Muskels liegenden Hautoberfläche ein Referenzmuster angeordnet werden, dessen Verformung über den optischen Sensor erfasst wird. Der optische Sensor kann z.B. ein CCD-Kamerasensor oder eine Matrix von Fotozellen sein.

In einer weiteren vorteilhaften Ausgestaltung weist das Außenohrmuskulaturerfassungsmittel wenigstens eine Lichtquelle und ein Lichterfassungsmittel auf. Hierbei kann die Lichtquelle auf der einen Seite der Ohrmuschel und das Lichterfassungsmittel auf der anderen Seite der Ohrmuschel angeordnet sein, so dass ein Lichtstrahl durch das Ohrmuschelgewebe geführt wird. Durch eine solche Durchleuchtung sind ebenfalls Muskelkontraktionen erfassbar. Bei einem kontrahierten Muskel erhöht sich die Gewebedichte im Vergleich zu einem nicht kontrahierten Muskel, so dass die am nicht Lichterfassungsmittel ankommende Lichtintensität abnimmt.

Die zuvor genannten Ausgestaltungen der Sensormittel und gegebenenfalls weitere Ausgestaltungen können in dem erfindungsgemäßen Außenohrmuskulaturerfassungsmittel auch in Kombination verwendet werden, wobei für den jeweils zu erfassenden Muskel das am Besten geeignete Sensorprinzip ausgewählt wird.

Gemäß einer vorteilhaften Weiterbildung der Erfindung weist das Außenohrmuskulaturerfassungsmittel eine erste Schnittstelleneinrichtung zur drahtlosen Datenübertragung zur Ausgabe erfasster Muskelsignale an die Steuereinrichtung auf. Die Schnittstelleneinrichtung kann beispielsweise als Bluetooth-Schnittstelle oder vergleichbare Funkstelle ausgebildet sein. Über die Schnittstelleneinrichtung können die erfassten Signale der äußeren Ohrmuskulatur an die Steuereinrichtung abgegeben werden, die die Signale verarbeitet und beispielsweise eine Prothese steuert. Die drahtlose Schnittstelle ermöglicht es, das Außenohrmuskulaturerfassungsmittel kompakt bauend auszuführen und auf eine als lästig empfundene Kabelverbindung zwischen dem Außenohrmuskulaturerfassungsmittel und der Steuereinrichtung verzichten zu können.

Das Außenohrmuskulaturerfassungsmittel kann vorteilhaft als reines Erfassungsmittel, d.h. als Sensorsystem ausgebildet sein, und keine weiteren Baueinheiten beispielsweise für eine Prothesensteuerung enthalten. Vorteilhaft ist auch eine integrierte Ausbildung des Außenohrmuskulaturerfassungsmittels und der Steuereinrichtung als eine Einheit. In einer vorteilhaften Ausgestaltung der Erfindung ist das Außenohrmuskulaturerfassungsmittel integriert mit einer anderen Vorrichtung ausgebildet, die auch für andere Funktionen eingerichtet ist. Vorteilhaft ist eine am Ohr eines Lebewesens zu tragende Vorrichtung, die wenigstens ein Außenohrmuskulaturerfassungsmittel nach dem zuvor beschriebenen Prinzip sowie ein weiteres Funktionsmittel zur Ausführung einer weiteren, nicht zur Erfassung der Ohrmuskulatur eingerichteten Funktion aufweist. Das weitere Funktionsmittel kann beispielsweise eine Hörhilfe sein. So kann das Außenohrmuskulaturerfassungsmittel in ein am Ohr zu tragendes Hörgerät integriert werden. Auch eine Integration oder Befestigung des Außenohrmuskulaturerfassungsmittels an einem Brillenbügel, einer am Ohr zu tragenden Bluetooth-Freisprecheinrichtung oder einem Kopfhörer ist vorteilhaft. Eine vorteilhafte Hilfseinrichtung für ein Lebewesen weist ein Außenohrmuskulaturerfassungsmittel nach dem zuvor beschriebenen Prinzip und eine damit verbundene Steuereinrichtung auf. Die Steuereinrichtung ist zur Verarbeitung der von dem Außenohrmuskulaturerfassungsmittel erfassten Signale und zur Ausgabe von Steuerungssignalen an ein Hilfsmittel eingerichtet. Die Hilfseinrichtung kann als kompakte, im Bereich des Ohrs eines Lebewesens anzubringende Vorrichtung ausgebildet sein, bei der das Außenohrmuskulaturerfassungsmittel und die Steuereinrichtung integriert ausgebildet sind. Die Steuereinrichtung kann auch separat am Körper des Lebewesens angeordnet sein. Das Hilfsmittel kann vorteilhaft ein Unterstützungsmittel für einen behinderten Menschen sein, z.B. eine Prothese, ein Rollstuhl oder ein Sprachcomputer. Vorteilhaft kann das Hilfsmittel auch ein sonstiges Gerät des täglichen Lebens sein, wie z.B. ein Telefon, ein Kopfhörer oder Computerspiel.

Vorteilhaft können die von dem Außenohrmuskulaturerfassungsmittel erfassten Muskelsignale auch zur Steuerung von Haushaltsgeräten, Aufzügen oder sonstigen von Menschen zu bedienenden Geräten verwendet werden, wie z.B. Computer oder Musikinstrumente. Wie erkennbar ist, ist die Erfindung von grundlegender Art und erlaubt einen sehr weiten Einsatzbereich. Mit der Erfindung ist eine Unterstützung sowohl von behinderten als auch von nicht behinderten Personen vorteilhaft möglich.

Gemäß einer vorteilhaften Weiterbildung der Erfindung weist die Hilfseinrichtung eine zweite Schnittstelleneinrichtung zur drahtlosen Datenübertragung zur Ausgabe der Steuerungssignale an das Hilfsmittel auf. Die drahtlose zweite Schnittstelleneinrichtung kann beispielsweise als Funkschnittstelle, z.B. Bluetooth, ausgebildet sein. Hierdurch wird wiederum der erforderliche Verdrahtungsaufwand verringert.

Vorteilhaft handelt es sich bei dem durch die Steuerungssignale steuerbaren Hilfsmittel um ein durch elektrische Signale steuerbares Hilfsmittel.

Gemäß einer vorteilhaften Weiterbildung der Erfindung ist das Außenohrmuskulaturerfassungsmittel teilweise oder vollständig in dem Lebewesen implantierbar ausgebildet. Im Falle einer teilweisen Implantierbarkeit ist es vorteilhaft, zumindest die Elektroden zur Erfassung der Aktivierung der Ohrmuskulatur implantierbar auszuführen. Gemäß einer vorteilhaften Weiterbildung der Erfindung ist die gesamte Hilfseinrichtung, inklusive der Steuereinrichtung, als in das Lebewesen implantierbares System ausgebildet.

Gemäß einer vorteilhaften Weiterbildung der Erfindung ist die Hilfseinrichtung zur Ausgabe von Steuerungssignalen an ein Cochlea-Implantat eingerichtet. Die Hilfseinrichtung ist hierbei bezüglich der Steuerungssignale kompatibel zum Cochlea-Implantat ausgebildet, so dass ein Cochlea-Implantat, das die Steuerungssignale der Hilfseinrichtung empfängt, gesteuert werden kann. Beispielsweise kann das Cochlea-Implantat durch die Steuerungssignale in unterschiedliche Betriebsmodi gebracht werden, z.B. von einem Normalmodus in einen Telefonmodus, der es einem Benutzer des Cochlea-Implantats erleichtert, auch bei störenden Umgebungsgeräuschen effektiv zu telefonieren. Hierbei kann insbesondere das Empfangsfrequenzband des Cochlea-Implantats an das von einem Telefon übertragene Sprachband angepasst werden, so dass Umgebungsgeräusche außerhalb des Sprachbands ausgeblendet werden.

Gemäß einer vorteilhaften Weiterbildung der Erfindung ist die Hilfseinrichtung zur Ausgabe von Steuerungssignalen an eine Armprothese eingerichtet. Dies erlaubt es einem die Hilfseinrichtung benutzenden Lebewesen, durch Aktivierung der äußeren Ohrmuskulatur, die Armprothese zu steuern.

Die Erfindung wird nachfolgend anhand eines Ausführungsbeispiels unter Verwendung einer Zeichnung näher erläutert.

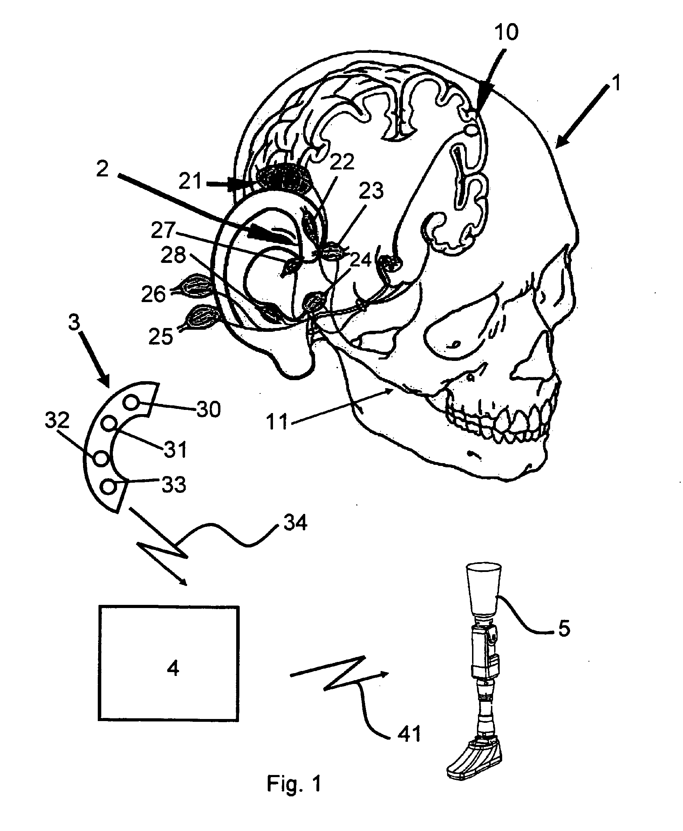

Die Figur 1 zeigt eine Hilfseinrichtung für einen Menschen mit einem am Außenohr angeordneten Außenohrmuskulaturerfassungsmittel.

Die Figur 1 zeigt den Kopf 1 eines Menschen, ein Ohr 2, ein Außenohrmuskulaturerfassungsmittel 3, eine Steuereinrichtung 4 sowie als Hilfsmittel eine Beinprothese 5. Bezüglich des Kopfs 1 sind zudem das motokortikale Repräsentationsareal 10 sowie der Nervus fazialis 11 dargestellt. In Bezug auf das Außenohr 2 sind beispielhaft Muskeln 21, 22, 23, 24, 25, 26, 27, 28 als Teile der äußeren Ohrmuskulatur eines Menschen dargestellt.

Zur Erfassung einer Aktivierung zumindest von Teilen der äußeren Ohrmuskulatur ist das Außenohrmuskulaturerfassungsmittel 3 vorgesehen. Das Außenohrmuskulaturerfassungsmittel 3 ist beispielsweise in Form eines am Außenohr zu tragenden Clips ausgestaltet, z.B. in der Form vergleichbar mit einem Hörgerät. Das Außenohrmuskulaturerfassungsgerät weist Sensormittel 30, 31, 32, 33 auf, über die in diesem Ausführungsbeispiel vier der zuvor erwähnten Muskeln 21, 22, 23, 24, 25, 26, 27, 28 erfasst werden. Das Außenohrmuskulaturerfassungsmittel 3 weist zudem eine Batterie zur Stromversorgung, einen Mehrkanal-EMG-Verstärker zur Verstärkung der Signale der Sensormittel 30, 31, 32, 33, ggf. Analog/Digital-Wandler zur Digitalisierung der Signale und einen Mikroprozessor zur Verarbeitung der Signale auf. Zudem ist eine Schnittstelleneinrichtung zur drahtlosen Datenübertragung vorgesehen, mit der über eine Datenübertragungsstrecke 34 Daten zur Steuereinrichtung 4 übertragen werden.

Die Steuereinrichtung 4 verarbeitet die empfangenen Daten von dem Außenohrerfassungsmittel 3 und bestimmt daraus Steuerungssignale zur Steuerung der Beinprothese 5. Die Steuerungssignale werden im Ausführungsbeispiel über eine weitere drahtlose Schnittstelleneinrichtung über eine Datenübertragungsstrecke 41 an die Beinprothese 5 übertragen.

Der Anwender kann nun durch willkürliche Aktivierung bestimmter Teile der äußeren Ohrmuskulatur die Beinprothese 5 nach Wunsch steuern.

## Patentansprüche

1. Hilfseinrichtung für ein Lebewesen mit einer Steuereinrichtung (4), die zur Ausgabe von Steuerungssignalen an ein technisches Hilfsmittel (5) eingerichtet ist, über die das technische Hilfsmittel (5) steuerbar ist, **dadurch gekennzeichnet, dass** die Hilfseinrichtung ein Außenohrmuskulaturerfassungsmittel (3) aufweist, das eine Aktivierung zumindest eines Teils der äußeren Ohrmuskulatur (21 bis 28) eines Lebewesens erfasst, wobei die Steuereinrichtung (4) mit dem Außenohrmuskulaturerfassungsmittel (3) verbunden ist, wobei die Steuereinrichtung (4) zur Verarbeitung der von dem Außenohrmuskulaturerfassungsmittel (3) erfassten Signale und zur Ausgabe von Steuerungssignalen an das technische Hilfsmittel (5) eingerichtet ist.

2. Hilfseinrichtung nach Anspruch 1, **dadurch gekennzeichnet, dass** das Außenohrmuskulaturerfassungsmittel (3) ein Sensormittel (30 bis 33) zur Erfassung der elektrischen Muskelaktivität wenigstens eines Ohrmuskels (21 bis 28) aufweist.

3. Hilfseinrichtung nach einem der vorhergehenden Ansprüche, **dadurch gekennzeichnet, dass** das Außenohrmuskulaturerfassungsmittel (3) ein Sensormittel (30 bis 33) zur Erfassung einer durch einen Ohrmuskel (21 bis 28) ausgelösten mechanischen Bewegung aufweist.

4. Hilfseinrichtung nach einem der vorhergehenden Ansprüche, **dadurch gekennzeichnet, dass** das Außenohrmuskulaturerfassungsmittel (3) wenigstens eine Lichtquelle und ein Lichterfassungsmittel aufweist.

5. Hilfseinrichtung nach einem der vorhergehenden Ansprüche, **dadurch gekennzeichnet, dass** das Außenohrmuskulaturerfassungsmittel (3) eine erste Schnittstelleneinrichtung zur drahtlosen Datenübertragung zur Ausgabe erfasster Muskelsignale an die Steuereinrichtung (4) aufweist.

6. Hilfseinrichtung nach Anspruch 1, **dadurch gekennzeichnet, dass** das Hilfsmittel (5) ein Unterstützungsmittel für einen behinderten Menschen ist, insbesondere eine Prothese, ein Rollstuhl oder ein Sprachcomputer.

7. Hilfseinrichtung nach Anspruch 6, **dadurch gekennzeichnet, dass** die Hilfseinrichtung eine zweite Schnittstelleneinrichtung zur drahtlosen Datenübertragung zur Ausgabe der Steuerungssignale an das Hilfsmittel (5) aufweist.

8. Hilfseinrichtung nach einem der vorhergehenden Ansprüche, **dadurch gekennzeichnet, dass** das Außenohrmuskulaturerfassungsmittel teilweise oder vollständig in einem Lebewesen implantierbar ausgebildet ist.

9. Hilfseinrichtung nach einem der vorhergehenden Ansprüche, **dadurch gekennzeichnet, dass** die Hilfseinrichtung zur Ausgabe von Steuerungssignalen an ein Cochlea-Implantat eingerichtet ist, wobei die Steuerungssignale zum Cochlea-Implantat kompatible Steuerungssignale sind.

10. Hilfseinrichtung nach einem der vorhergehenden Ansprüche, **dadurch gekennzeichnet, dass** die Hilfseinrichtung zur Ausgabe von Steuerungssignalen an eine Armprothese eingerichtet ist, wobei die Steuerungssignale zur Armprothese kompatibel sind.

## Claims

1. An auxiliary device for a living being with a control device (4), which is arranged to output control signals to a technical aid (5), by means of which the technical aid (5) can be controlled, **characterized in that** the auxiliary device comprises an outer ear musculature detection means (3) which detects an activation of at least part of the outer ear musculature (21 to 28) of a living being, wherein the control device is connected to the outer ear musculature detection means (3), wherein the control device (4) is arranged to process the signals detected by the outer ear musculature detection means (3) and to output control signals to the technical aid (5).

2. The auxiliary device as claimed in claim 1, **characterized in that** the outer ear musculature detection means (3) has a sensor means (30 to 33) for detecting the electric muscle activity of at least one ear muscle (21 to 28).

3. The auxiliary device as claimed in one of the preceding claims, **characterized in that** the outer ear musculature detection means (3) has a sensor means (30 to 33) for detecting a mechanical movement triggered by an ear muscle (21 to 28).

4. The auxiliary device as claimed in one of the preceding claims, **characterized in that** the outer ear musculature detection means (3) has at least one light source and a light detection means.

5. The auxiliary device as claimed in one of the preceding claims, **characterized in that** the outer ear musculature detection means (3) has a first interface device for wireless data transmission for outputting detected muscle signals to the control device (4).

6. The auxiliary device as claimed in claim 1, **characterized in that** the aid (5) is a support means for a disabled person, more particularly a prosthesis, a wheelchair or a voice computer.

7. The auxiliary device as claimed in claim 6, **characterized in that** the auxiliary device has a second interface device for wireless data transmission for the outputting control signals to the aid (5).

8. The auxiliary device as claimed in one of the preceding claims, **characterized in that** the outer ear musculature detection means is designed such that it can be partly or wholly implanted into a living being.

9. The auxiliary device as claimed in one of the preceding claims, **characterized in that** the auxiliary device is embodied to output control signals to a cochlear implant, with the control signals being control signals that are compatible with the cochlear implant.

10. The auxiliary device as claimed in one of the preceding claims, **characterized in that** the auxiliary device is embodied to output control signals to an arm prosthesis, with the control signals being compatible with the arm prosthesis.

## Revendications

1. Dispositif auxiliaire pour un être vivant, doté d'un dispositif de commande (4) conçu pour délivrer à un accessoire technique (5) des signaux de commande qui permettent de commander l'accessoire technique (5), **caractérisé en ce que**
le dispositif auxiliaire présente un moyen (3) de saisie de la musculature de l'oreille externe qui permet d'activer au moins une partie de la musculature externe (21 à 28) de l'oreille d'un être vivant,
**en ce que** le dispositif de commande (4) est relié au moyen (3) de saisie de la musculature de l'oreille externe et
**en ce que** le dispositif de commande (4) est conçu pour traiter les signaux saisis par le moyen (3) de saisie de la musculature de l'oreille externe et pour délivrer des signaux de commande à l'accessoire technique (5).

2. Dispositif auxiliaire selon la revendication 1, **caractérisé en ce que** le moyen (3) de saisie de la musculature de l'oreille externe présente un moyen de détection (30 à 33) qui détecte l'activité électrique musculaire d'au moins un muscle (21 à 28) de l'oreille.

3. Dispositif auxiliaire selon l'une des revendications précédentes, **caractérisé en ce que** le moyen (3) de saisie de la musculature de l'oreille externe présente un moyen de détection (30 à 33) qui détecte un déplacement mécanique provoqué par un muscle (21 à 28) de l'oreille.

4. Dispositif auxiliaire selon l'une des revendications précédentes, **caractérisé en ce que** le moyen (3) de saisie de la musculature de l'oreille externe présente au moins une source de lumière et un moyen de détection de la lumière.

5. Dispositif auxiliaire selon l'une des revendications précédentes, **caractérisé en ce que** le moyen (3) de saisie de la musculature de l'oreille externe présente un premier dispositif d'interface qui transmet sans fil au dispositif de commande (4) les données pour l'émission des signaux musculaires saisis.

6. Dispositif auxiliaire selon la revendication 1, **caractérisé en ce que** l'accessoire (5) est un moyen d'assistance pour personne handicapée, notamment une prothèse, une chaise roulante ou un ordinateur à commande vocale.

7. Dispositif auxiliaire selon la revendication 6, **caractérisé en ce que** l'accessoire présente un deuxième dispositif d'interface qui transmet sans fil les données pour l'émission des signaux de commande à l'accessoire (5).

8. Dispositif auxiliaire selon l'une des revendications précédentes, **caractérisé en ce que** le moyen de saisie de la musculature de l'oreille externe peut être implanté totalement ou partiellement dans un être vivant.

9. Dispositif auxiliaire selon l'une des revendications précédentes, **caractérisé en ce que** l'accessoire est conçu pour délivrer des signaux de commande à un implant cochléaire, les signaux de commande étant des signaux de commande compatibles avec l'implant cochléaire.

10. Dispositif auxiliaire selon l'une des revendications précédentes, **caractérisé en ce que** l'accessoire est conçu pour délivrer des signaux de commande à une prothèse du bras, les signaux de commande étant compatibles avec la prothèse du bras.
